# EUROPEAN PATENT APPLICATION

(11) **EP 3 730 623 A1**
(43) Date of publication of application: **28.10.2020**
(21) Application number: 19842856.7
(22) Date of filing: 25.12.2019
(51) Int. Cl.: C12P 19/26, C12P 19/14

(54) **SMALL-MOLECULE HYALURONIC ACID OR SALT THEREOF, AND PREPARATION METHOD THEREFOR**

(30) Priority: 05.03.2019 CN 201910164881
(71) Applicant: Shandong Awa Biopharm Co., Ltd., Binzhou City, Shandong 256600 (CN)
(72) Inventor: HAN, Xiuyun, Binzhou City, Shandong 256600 (CN)
(74) Representative: Adamson Jones
(86) International application number: PCT/CN2019/128316
(87) International publication number: WO 2020/177455

(57) **Abstract**

The present disclosure relates to the technical field of bioengineering, and specifically provides small-molecule hyaluronic acid or salts thereof and a preparation method thereof. In the present disclosure, *Lactobacillus plantarum,* with the accession number of preservation of the strain: CGMCC No. 16836, is fermented to produce a hyaluronidase solution, the hyaluronidase solution enzymatically hydrolyzes hyaluronic acid having a molecular weight of 500 kDa-700 kDa or salts thereof, and the prepared small-molecular hyaluronic acid or salts thereof has/have a molecular weight of 1 kDa-60 kDa; and the small-molecule hyaluronic acid enzymolysis liquid is subjected to enzyme inactivation, adsorption by activated carbon for impurity removal, filtration by a microporous filter membrane, and low-temperature spray drying to obtain small-molecule hyaluronic acid or salts thereof. The product obtained in the present disclosure has high safety and good transdermal absorption, greatly ensures the integrity of molecular structure and biological activity, and thus has broader application.

## Description

### Cross-reference to Related Applications

The present disclosure claims the priority of the Chinese patent application No. CN201910164881.3, filed with the Chinese Patent Office on March 5, 2019 and entitled "Method for Preparing Small-molecule Hyaluronic Acid or Salts Thereof", which is incorporated herein by reference in its entirety.

### Technical Field

The present disclosure relates to the technical field of bioengineering, and particularly to small-molecule hyaluronic acid or salts thereof and a preparation method thereof.

### Background Art

Hyaluronic acid (HA), which is an acid mucopolysaccharide, is an unbranched high-molecule glycosaminoglycan composed of disaccharide repeating units of N-acetylglucosamine and D-glucuronic acid, is present in intercellular matrix of animal tissues and capsules of some bacteria, and is widely used in the industry sectors such as cosmetics, food and medicine.

Recent studies have shown that small-molecule hyaluronic acid exhibits very strong biological activity (Hui ZM, a bioactive hyaluronic acid fragment, its manufacture method, its formulation and its applications, Patent number 2014101553593.5.), has the functions of improving immune function of skin, eliminating skin inflammation, promoting wound healing, treating acute and chronic pharyngitis, treating gingivitis, etc., which is an activator of immune cells and cytokines. Small-molecule hyaluronic acid has good permeability and absorption, can be absorbed by the dermis and intestinal tract very easily, and can quickly replenish the body's loss of hyaluronic acid. Therefore, small-molecule hyaluronic acid has broad application prospects in the fields of food health care, cosmetics and clinical medicine.

In recent years, with the promotion of the research and application of small-molecule hyaluronic acid, attention has been paid to the research on the degradation of HA and the preparation of its degradation products at home and abroad. At present, there are mainly three degradation methods: physical degradation, chemical degradation and biological degradation. Physical degradation methods mainly include heating, mechanical shear force, ultraviolet ray, ultrasonic wave, 60C irradiation, γ-ray irradiation and other factors, which can all degrade HA. However, the molecular weight resulting from preparation by a physical method is higher than 10,000, the resulting molecular weight is distributed in a relatively large range, and the stability of the product is relatively poor. The chemical degradation methods of HA include hydrolysis and oxidative degradation. Hydrolysis includes acid hydrolysis (HCL) and alkaline (NaOH) hydrolysis. The common oxidizing agents for oxidative degradation are sodium hypochlorite and hydrogen peroxide. For the chemical degradation methods, the relative molecular mass of the degradation products can be controlled by varying the addition amount of acid, alkali or oxidizing agents and the reaction time, and the degradation cost is relatively low, which facilitates mass production. However, chemical degradation methods introduce chemical agents, have complex reaction conditions, are likely to affect the properties of HA and bring forth difficulties to the purification of the product, and produce a large amount of industrial wastewater. Bio-enzyme degradation is caused by the breakage of β-glycosidic bond under the action of hyaluronidase. Enzymatic degradation is mild and is the best technical means for preparing small-molecule hyaluronic acid. Hyaluronidase, also known as hyalase, is a glycosidase that can cause HA to be reduced in molecular weight, can also degrade acid mucopolysaccharides in other tissues, is widely found in eukaryotes and prokaryotes, and has specific physiological activities.

For example, the invention patent with the application number CN201611127881.9 provides a method for preparing ultra-low molecular weight hyaluronic acid oligosaccharides and salts thereof by solid-liquid biphasic enzymolysis combined with ultrafiltration, in which enzymolysis of hyaluronic acid is realized by the enzymic method. However, the current hyaluronidase is usually extracted and prepared from animal tissues, which is expensive and is not suitable for industrial application. Therefore, a method for preparing small-molecule hyaluronic acid with a low cost, a simple process and suitable for industrial large-scale production has important application potential.

Moreover, the extraction process for hyaluronic acid is mainly organic reagent precipitation method, which causes environmental pollution, has high energy consumption and has low extraction yield of small-molecule hyaluronic acid. In addition, extraction of hyaluronic acid is also carried out by spray drying, but conventional spray dryers have an inlet air temperature of 180 °C or above, and for heat-sensitive materials, the physical properties or chemical properties will easily change, especially the bioactive materials, such as bacteria, active enzymes, etc., which will be inactivated.

### Summary

The objects of the present disclosure include, for example, providing small-molecule hyaluronic acid or salts thereof and a preparation method thereof to overcome the above defects.

In the method provided by the embodiments of the present disclosure, small-molecule hyaluronic acid or salts thereof is/are mainly obtained by degradation by using hyaluronidase produced by *Lactobacillus plantarum* in combination with low-temperature spray drying.

The embodiments of the present disclosure may be implemented, for example, in the following manner:
A method for preparing small-molecule hyaluronic acid or salts thereof according to an embodiment of the present disclosure comprises the following steps:
(1) preparation of enzymolysis reaction liquid: adding hyaluronidase to purified water, starting stirring, adding a solid powder of hyaluronic acid or salts thereof slowly, and obtaining enzymolysis liquid by enzymolysis reaction, wherein the hyaluronidase is produced by fermentation of a *Lactobacillus plantarum* mutant strain CnT012-56; the taxonomic denomination of the strain is *Lactobacillus plantarum,* the preservation institution is China General Microbiological Culture Collection Center (CGMCC), the address is NO.1 West Beichen Road, Chaoyang District, Beijing, the date of preservation is November 28, 2018, and the accession number of preservation is CGMCC No. 16836;
(2) inactivation of hyaluronidase: performing a heating treatment to the enzymolysis liquid of step (1) to denature and inactivate the hyaluronidase in the enzymolysis liquid;
(3) adsorption: adsorbing the enzymolysis liquid in step (2) to obtain a mixture liquid;
(4) filtration and purification: removing an adsorbent and denatured hyaluronidase from the mixture liquid in step (3), and filtering to obtain a solution of pure small-molecule hyaluronic acid and salts thereof; and
(5) low-temperature spray drying: making the solution of small-molecule hyaluronic acid and salts thereof in step (4) subject to low-temperature spray drying to obtain a solid powder of small-molecule hyaluronic acid and salts thereof having a desired molecular weight.

Optionally, in the above-described method for preparing small-molecule hyaluronic acid or salts thereof, in step (1), hyaluronidase is first added to purified water, stirring is started, the pH is adjusted to 5-8, the temperature is 30-40 °C, a solid powder of hyaluronic acid or salts thereof is slowly added thereto so that the mass volume concentration reaches 50-100 g/L, 0.5-1.5 h after the degradation reaction, the solid powder of hyaluronic acid or salts thereof continues to be added slowly so that the mass volume concentration reaches 100-200 g/L, and enzymolysis continues for 5-8 h.

Optionally, in the above-described method for preparing small-molecule hyaluronic acid or salts thereof, in step (1), the addition amount of the hyaluronidase is 10²-10⁵ IU per gram of hyaluronic acid or salts thereof.

Optionally, in the above-described method for preparing small-molecule hyaluronic acid or salts thereof, in step (2), the condition of denaturation and inactivation of the hyaluronidase is: heating the enzymolysis liquid to 45-80 °C and maintaining for 5-60 min.

Optionally, in the above-described method for preparing small-molecule hyaluronic acid or salts thereof, in step (3), the adsorption condition of the enzymolysis liquid is: 0.01%-1% activated carbon, and adsorption for 30-90 min, and in the step (4), a cellulose filter membrane having a pore diameter of 0.22 µm is used for filtration.

Optionally, in the above-described method for preparing small-molecule hyaluronic acid or salts thereof, hyaluronate is sodium hyaluronate, and hydrochloric acid or sodium hydroxide solution is used for the pH adjustment.

Optionally, in the above-described method for preparing small-molecule hyaluronic acid or salts thereof, in step (4), the final concentration of the solution of small-molecule hyaluronic acid or salts thereof is 10%-20%.

Optionally, in the above-described method for preparing small-molecule hyaluronic acid or salts thereof, in step (5), the condition of the low-temperature spray drying method is: an inlet air temperature of 40 °C-80 °C, an outlet air temperature of 40 °C, and a feed rate of 100 mL/h-300 mL/h.

Optionally, in the above-described method for preparing small-molecule hyaluronic acid or salts thereof, the hyaluronic acid or salts thereof used for enzymolysis in step (1) has/have a molecular weight of 500 kDa-700 kDa, and the small-molecule hyaluronic acid or salts thereof prepared in step (5) has/have a molecular weight of 1 kDa-60 kDa.

Optionally, in the above-described method for preparing small-molecule hyaluronic acid or salts thereof, the method for producing the hyaluronidase comprises the steps of:
(a) inoculating a *Lactobacillus plantarum* CnT012-56 strain into a sterilized seed medium, with a culture temperature of 30 °C-37 °C, a rotation speed of 0-100 rpm, and a culture duration of 8-24 h, to obtain a seed culture solution;
(b) inoculating the seed culture solution in step (1) into a sterilized fermentation medium, with a culture temperature of 30 °C-37 °C, a rotation speed of 0-150 rpm, and a culture duration of 20-48 h, to obtain a hyaluronidase fermentation broth;
(c) centrifuging the hyaluronidase fermentation broth in step (2) to obtain a supernatant;
(d) ultrafiltering the hyaluronidase supernatant in step (3) using an ultrafiltration membrane having a pore diameter with molecular weight cutoff of 200 kDa, to obtain a filtrate; and
(e) ultrafiltering the filtrate in step (4) using an ultrafiltration membrane having a pore diameter with molecular weight cutoff of 20 kDa, to remove small-molecule impurities to obtain purified hyaluronidase.

Optionally, in the above-described method for preparing small-molecule hyaluronic acid or salts thereof, the components of the seed medium in step (a) are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with the pH of 6.5.

Optionally, in the above-described method for preparing small-molecule hyaluronic acid or salts thereof, the components of the fermentation medium in step (b) are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with the pH of 6.5.

The small-molecule hyaluronic acid or salts thereof provided by the embodiments of the present disclosure is/are prepared by the above-described method.

The advantageous effects of the embodiments of the present disclosure include, for example:
(1) The embodiments of the present disclosure provide establishment of a hyaluronic acid enzymolysis reaction system. Hyaluronidase involved in the embodiments of the present disclosure is derived from *Lactobacillus plantarum.* As a kind of probiotics, *Lactobacillus plantarum* can be used in food, and the produced hyaluronidase has high safety, and small-molecule hyaluronic acid or salts thereof prepared by degradation therefrom is/are safe and reliable, and very suitable for the use in food, cosmetics and pharmaceutical industries.
(2) The embodiments of the present disclosure further provide a method for inactivating and purifying enzymolysis liquid, wherein hyaluronidase inactivating method is heating enzymolysis liquid to cause the hyaluronidase to be denatured and inactivated, and enzymolysis liquid purifying method is adsorbing enzymolysis liquid using activated carbon first, and then filtering enzymolysis liquid using a cellulose filter membrane. The method is convenient and efficient, and realizes efficient inactivation and purification.
(3) The embodiments of the present disclosure further provide a method for preparing a solid powder of small-molecule hyaluronic acid, i.e., the low-temperature spray drying method. Compared with the traditional alcohol precipitation method, the method has the advantages of high yield, no pollution, low energy consumption and no residual organic solvent; moreover, compared with the traditional drying method, the spray drying in this method is completed at a material temperature of lower than 80 °C, and the inlet air temperature thereof is at least about 40 °C, and the preparation of the small-molecule hyaluronic acid using this method preserves, to the maximum extent, the structural integrity and biological activity of molecules.
(4) Moreover, the small-molecule hyaluronic acid prepared by the method of the embodiments of the present disclosure has good quality, and the whiteness of the solid powder of hyaluronic acid is 93% or above, the moisture content can be less than 6.3%, and the molecular weight is 1 kDa-60 kDa; the purity of the small-molecule hyaluronic acid is high, the content can reach 97% or above; and the yield is high, which can be as high as 90%.

### Detailed Description of Embodiments

In order to enable a person skilled in the art to get a better understanding of the technical solutions of the present disclosure, the technical solutions will be described clearly and completely below with reference to the embodiments of the present disclosure. Apparently, the embodiments described are merely some of the embodiments of the present disclosure, rather than all of the embodiments. All the other embodiments that are obtained by a person of ordinary skills in the art without inventive effort on the basis of the embodiments of the present disclosure shall be covered by the protection scope of the present disclosure.

The hyaluronidase used in the embodiments of the present disclosure is derived from *Lactobacillus plantarum.* Optionally, the *Lactobacillus plantarum* is *Lactobacillus plantarum* CnT012-56 obtained by the following process: obtaining a *Lactobacillus plantarum* strain producing hyaluronidase by isolation from bacteria-infected hyaluronic acid fermentation broth, and then carrying out mutation breeding by using a multi-functional plasma mutagenesis system to obtain a *Lactobacillus plantarum* CnT012-56 strain with high hyaluronidase yield.

The strain was preserved in China General Microbiological Culture Collection Center (CGMCC), with the date of preservation: November 28, 2018, and the accession number of preservation: CGMCC No. 16836; and identified as *Lactobacillus plantarum,* with the denomination of *Lactobacillus plantarum* CnT012-56. When the strain is cultured in a fermentation medium for 38 h, hyaluronidase in its fermentation broth has the highest activity, which can reach 6000 IU/mL. After the fermentation broth is subjected to thallus removal by centrifugation, impurity removal by a microfiltration membrane, and purification and concentration by ultrafiltration, the activity can reach 70000 IU/mL.

Optionally, a method for producing hyaluronidase using the *Lactobacillus plantarum* mutant strain of the embodiments of the present disclosure comprises the steps of:
(1) inoculating a *Lactobacillus plantarum* CnT012-56 strain into a sterilized seed medium, with a culture temperature of 30 °C -37 °C, a rotation speed of 0-100 rpm, and a culture duration of 8-24 h, to obtain a seed culture solution;
(2) inoculating the seed culture solution into a sterilized fermentation medium, with a culture temperature of 30 °C -37 °C, a rotation speed of 0-150 rpm, and a culture duration of 20-48 h, to obtain a hyaluronidase fermentation broth;
(3) centrifuging the hyaluronidase fermentation broth to obtain a supernatant;
(4) ultrafiltering the hyaluronidase supernatant using an ultrafiltration membrane having a pore diameter with molecular weight cutoff of 200 kDa, to obtain a filtrate; and
(5) ultrafiltering the filtrate using an ultrafiltration membrane having a pore diameter with molecular weight cutoff of 20 kDa, to remove small-molecule impurities to obtain purified hyaluronidase.

Optionally, the components of the seed medium are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with the pH of 6.5; and optionally, the components of the fermentation medium are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with the pH of 6.5.

Optionally, in the embodiments of the present disclosure, the method for determination of the molecular weight of the small-molecule hyaluronic acid or salts thereof is an intrinsic viscosity method assay (WANG Yanhou, WANG Fengshan, GUO Xueping, et al. Study on Determination Method of Intrinsic Viscosity of Sodium Hyaluronate [J]. Chinese Pharmaceutical Journal, 2004, 39 (6): 469-471).

Optionally, in the embodiments of the present disclosure, the whiteness, moisture content, and purity content are determined according to "QB/T 4416-2012 cosmetic ingredients sodium hyaluronate".

Optionally, the method for preparing small-molecule hyaluronic acid or salts thereof provided by an embodiment of the present disclosure comprises the following steps: (1) preparation of enzymolysis reaction liquid: adding hyaluronidase to purified water, starting stirring, adding a solid powder of hyaluronic acid or salts thereof slowly, and obtaining enzymolysis liquid by enzymolysis reaction; (2) inactivation of hyaluronidase: performing a heating treatment to the enzymolysis liquid of step (1) to denature and inactivate the hyaluronidase in the enzymolysis liquid; (3) adsorption: adsorbing the enzymolysis liquid in step (2) to obtain a mixture liquid; (4) filtration and purification: removing an adsorbent and denatured hyaluronidase from the mixture liquid in step (3), and filtering to obtain a solution of pure small-molecule hyaluronic acid and salts thereof; and (5) low-temperature spray drying: making the solution of small-molecule hyaluronic acid and salts thereof in step (4) subject to low-temperature spray drying to obtain a solid powder of small-molecule hyaluronic acid and salts thereof having a desired molecular weight.

Optionally, in the above-described method for preparing small-molecule hyaluronic acid or salts thereof, in step (1), the solid powder of hyaluronic acid or salts thereof is added in a stepwise manner, which can ensure that the solid powder of hyaluronic acid or salts thereof is dissolved and reacts with hyaluronidase, thereby ensuring the effect of enzymolysis.

Optionally, in the above-described method for preparing small-molecule hyaluronic acid or salts thereof, in step (1), the addition amount of the hyaluronidase is 10²-10⁵ IU per gram of hyaluronic acid or salts thereof, and the main reason for adopting this ratio is that an excessively high addition amount of hyaluronidase will result in excessively rapid enzymolysis of hyaluronic acid or salts thereof, resulting in non-uniform molecular weight of the product, and an excessively low addition amount of hyaluronidase cannot ensure the effect of enzymolysis.

Optionally, in the above-described method for preparing small-molecule hyaluronic acid or salts thereof, filtration using a cellulose filter membrane having a pore diameter of 0.22 µm in step (4) can filter out the activated carbon and hyaluronidase to the maximum extent, so as to ensure the purity of the subsequent product.

Optionally, in the above-described method for preparing small-molecule hyaluronic acid or salts thereof, hydrochloric acid or sodium hydroxide solution is used for adjusting pH, which ensures the safety of the final product on the one hand, and can reduce the production cost on the other hand.

Optionally, in the above-described method for preparing small-molecule hyaluronic acid or salts thereof, in step (4), the final concentration of the solution of small-molecule hyaluronic acid or salts thereof is 10%-20%, and this concentration is matched with the low-temperature spray drying method, ensuring the normal operation of the subsequent drying process.

The technical solutions of the present disclosure are further described below in connection with specific examples:

### Example 1

5 mL of hyaluronidase was added into 5 L of purified water, stirring was started, pH was adjusted to 5.0, the temperature was 30 °C, and 250 g of sodium hyaluronate with a molecular weight of 500 kDa was slowly added. The materials must be added slowly in order to be dissolved uniformly. Optionally, 30 min after the degradation reaction, another 250 g of sodium hyaluronate with a molecular weight of 500 kDa was added slowly. 5 h after the enzymolysis reaction, the molecular weight reached 60 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 40 °C, the outlet air temperature was 40 °C, and the flow rate was 100 mL/h. The finished solid powder of small-molecule hyaluronic acid has a whiteness of 95%, a moisture content of 6.3%, a molecular weight of 60 kDa, a hyaluronic acid content of 97%, and a yield of 89%.

### Example 2

6 mL of hyaluronidase was added into 5 L of purified water, stirring was started, pH was adjusted to 5.5, the temperature was 32 °C, and 300 g of sodium hyaluronate with a molecular weight of 650 kDa was slowly added. The materials must be added slowly in order to be dissolved uniformly. Optionally, 40 min after the degradation reaction, 400 g of sodium hyaluronate with a molecular weight of 650 kDa was then added slowly. 6 h after the enzymolysis reaction, the molecular weight reached 50 kDa, optionally, the enzymolysis liquid was heated to 50 °C and maintained for 50 min so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 50 °C, the outlet air temperature was 40 °C, and the flow rate was 100 mL/h. The finished solid powder of small-molecule hyaluronic acid has a whiteness of 96%, a moisture content of 7.3%, a molecular weight of 50 kDa, a hyaluronic acid content of 96%, and a yield of 90%.

### Example 3

7 mL of hyaluronidase was added into 5 L of purified water, stirring was started, pH was adjusted to 6.0, the temperature was 34 °C, and 400 g of sodium hyaluronate with a molecular weight of 700 kDa was slowly added. The materials must be added slowly in order to be dissolved uniformly. Optionally, 50 min after the degradation reaction, 300 g of sodium hyaluronate with a molecular weight of 700 kDa was then added slowly. 6.5 h after the enzymolysis reaction, the molecular weight reached 40 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 60 °C, the outlet air temperature was 40 °C, and the flow rate was 150 mL/h. The finished solid powder of small-molecule hyaluronic acid has a whiteness of 95%, a moisture content of 8.2%, a molecular weight of 40 kDa, a hyaluronic acid content of 97%, and a yield of 90%.

### Example 4

8 mL of hyaluronidase was added into 5 L of purified water, stirring was started, pH was adjusted to 6.5, the temperature was 36 °C, and 400 g of sodium hyaluronate with a molecular weight of 500 kDa was slowly added. The materials must be added slowly in order to be dissolved uniformly. Optionally, 60 min after the degradation reaction, 400 g of sodium hyaluronate with a molecular weight of 500 kDa was then added slowly. 7 h after the enzymolysis reaction, the molecular weight reached 30 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 65 °C, the outlet air temperature was 40 °C, and the flow rate was 100 mL/h. The finished solid powder of small-molecule hyaluronic acid has a whiteness of 93%, a moisture content of 7.2%, a molecular weight of 30 kDa, a hyaluronic acid content of 98%, and a yield of 91%.

### Example 5

8 mL of hyaluronidase was added into 5 L of purified water, stirring was started, pH was adjusted to 7.0, the temperature was 38 °C, and 400 g of sodium hyaluronate with a molecular weight of 650 kDa was slowly added. The materials must be added slowly in order to be dissolved uniformly. Optionally, 70 min after the degradation reaction, 400 g of sodium hyaluronate with a molecular weight of 650 kDa was then added slowly. 8 h after the enzymolysis reaction, the molecular weight reached 20 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 70 °C, the outlet air temperature was 40 °C, and the flow rate was 200 mL/h. The finished solid powder of small-molecule hyaluronic acid has a whiteness of 93%, a moisture content of 8.3%, a molecular weight of 20 kDa, a hyaluronic acid content of 98%, and a yield of 88%.

### Example 6

9 mL of hyaluronidase was added into 5 L of purified water, stirring was started, pH was adjusted to 8.0, the temperature was 40 °C, and 500 g of sodium hyaluronate with a molecular weight of 650 kDa was slowly added. The materials must be added slowly in order to be dissolved uniformly. Optionally, 80 min after the degradation reaction, 500 g of sodium hyaluronate with a molecular weight of 650 kDa was then added slowly. 7.5 h after the enzymolysis reaction, the molecular weight reached 10 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 75 °C, the outlet air temperature was 40 °C, and the flow rate was 250 mL/h. The finished solid powder of small-molecule hyaluronic acid has a whiteness of 93%, a moisture content of 7.8%, a molecular weight of 10 kDa, a hyaluronic acid content of 99%, and a yield of 89%.

### Example 7

10 mL of hyaluronidase was added into 5 L of purified water, stirring was started, pH was adjusted to 6.5, the temperature was 38 °C, and 400 g of sodium hyaluronate with a molecular weight of 700 kDa was slowly added. The materials must be added slowly in order to be dissolved uniformly. Optionally, 90 min after the degradation reaction, 500 g of sodium hyaluronate with a molecular weight of 700 kDa was then added slowly. 6 h after the enzymolysis reaction, the molecular weight reached 7 kDa-9 kDa, optionally, the enzymolysis liquid was heated to 50 °C and maintained for 1 h so that the hyaluronidase was denatured and inactivated. Optionally, 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. Optionally, the hyaluronic acid solution was subjected to low-temperature spray drying to prepare a solid powder of small-molecule hyaluronic acid. Optionally, the inlet air temperature of the low-temperature spray was 80 °C, the outlet air temperature was 40 °C, and the flow rate was 300 mL/h. The finished solid powder of small-molecule hyaluronic acid has a whiteness of 94%, a moisture content of 6.9%, a molecular weight of 1 kDa, a hyaluronic acid content of 99%, and a yield of 90%.

In addition, a solid powder of small-molecule hyaluronic acid was prepared using the conventional alcohol precipitation method instead of the low-temperature spray drying method of the embodiments of the present disclosure.

### Comparative Example 1

10 mL of hyaluronidase was added into 5 L of purified water, stirring was started, pH was adjusted to 6.5, the temperature was 38 °C, and 400 g of sodium hyaluronate with a molecular weight of 700 kDa was slowly added. The materials must be added slowly in order to be dissolved uniformly. 90 min after the degradation reaction, 500 g of sodium hyaluronate with a molecular weight of 700 kDa was then added slowly. 6 h after the enzymolysis reaction, the molecular weight reached 7 kDa-9 kDa, the enzymolysis liquid was heated to 50 °C and maintained for 1 h so that the hyaluronidase was denatured and inactivated. 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. The resultant pure hyaluronic acid solution was added to quadruple volume of alcohol for reverse precipitation, and stirring was started during the precipitation process. After the completion of precipitation, the solution was left standing for 1 h, the supernatant was removed, and one volume of alcohol was added for dehydration for three times, followed by centrifugation and supernatant removal, and then the solid was vacuum-dried to obtain a solid powder of small-molecular hyaluronic acid. The powder of small-molecule hyaluronic acid has a whiteness of 89%, a moisture content of 8.9%, a molecular weight of 9 kDa, a hyaluronic acid content of 95%, and a yield of 78%.

### Comparative Example 2

8 mL of hyaluronidase was added into 5 L of purified water, stirring was started, pH was adjusted to 6.5, the temperature was 36 °C, and 400 g of sodium hyaluronate with a molecular weight of 500 kDa was slowly added. The materials must be added slowly in order to be dissolved uniformly. 60 min after the degradation reaction, 400 g of sodium hyaluronate with a molecular weight of 500 kDa was then added slowly. 7 h after the enzymolysis reaction, the molecular weight reached 30 kDa, the enzymolysis liquid was heated to 50 °C and maintained for 1 h so that the hyaluronidase was denatured and inactivated. 5 g of activated carbon was added to the enzymolysis liquid for adsorption for 60 min, the enzymolysis liquid was filtered through a coarse filtration board, and then filtered through a cellulose filter membrane having a pore diameter of 0.22 µm to obtain a pure hyaluronic acid solution. The resultant pure hyaluronic acid solution was added to quadruple volumes of alcohol for reverse precipitation, and stirring was started during the precipitation process. After the completion of precipitation, the solution was left standing for 1 h, the supernatant was removed, and one volume of alcohol was added for dehydration for three times, followed by centrifugation and supernatant removal, and then the solid was vacuum-dried to obtain a solid powder of small-molecular hyaluronic acid. The powder of small-molecule hyaluronic acid has a whiteness of 87%, a moisture content of 9.2%, a molecular weight of 30 kDa, a hyaluronic acid content of 94%, and a yield of 79%.

As can be seen from Comparative Example 1 and Comparative Example 2, the product quality and product yield of the hyaluronic acid obtained by the conventional alcohol precipitation method are both inferior to those of the hyaluronic acid obtained by the low-temperature spray drying method.

Although the present disclosure has been described in detail by way of preferred examples, the present disclosure is not limited thereto. Various equivalent modifications or substitutions may be made by those of ordinary skills in the art to the examples of the present disclosure without departing from the spirit and substance of the present disclosure, which modifications or substitutions are intended to be within the scope of the present disclosure. Further, changes or replacements that would readily be conceivable to those skilled in the art within the technical scope disclosed by the present disclosure shall be covered by the scope of protection of the present disclosure. Therefore, the scope of protection of the present disclosure shall be determined by the scope of protection of the appended claims.

### Industrial Applicability

In the method for preparing small-molecule hyaluronic acid or salts thereof provided by the embodiments of the present disclosure, small-molecule hyaluronic acid or salts thereof is/are prepared by the steps such as enzymolysis, enzyme inactivation, adsorption, filtration, purification and low-temperature spray drying using hyaluronidase produced by *Lactobacillus plantarum.* The method has a simple technological process, and the hyaluronidase inactivation method used is convenient and efficient, which is conducive to the realization of high-efficiency inactivation and purification; the low-temperature spray drying used has the advantages of high yield, no pollution, low energy consumption and no residual organic solvent, and preserves, to the maximum extent, the structural integrity and biological activity of molecules; the resultant small-molecule hyaluronic acid has high whiteness and low moisture content, with a high content and a high yield, and can meet the requirements for different molecular weights; and *Lactobacillus plantarum* confers higher safety and lower production cost to the obtained product, effectively improving industrial production and application potential of small-molecule hyaluronic acid or salts thereof.

## Claims

1. A method for preparing small-molecule hyaluronic acid or salts thereof, **characterized by** comprising following steps:
(1) preparation of enzymolysis reaction liquid: adding hyaluronidase to purified water, starting stirring, adding a solid powder of hyaluronic acid or salts thereof slowly, and obtaining enzymolysis liquid by enzymolysis reaction, wherein the hyaluronidase is produced by fermentation of a *Lactobacillus plantarum* mutant strain CnT012-56; a taxonomic denomination of the strain is *Lactobacillus plantarum,* a preservation institution is China General Microbiological Culture Collection Center, with an address: No. 1, West Beichen Road, Chaoyang District, Beijing, a date of preservation: November 28, 2018, and an accession number of preservation: CGMCC No. 16836;
(2) inactivation of hyaluronidase: performing a heating treatment to the enzymolysis liquid of step (1), so as to denature and inactivate the hyaluronidase in the enzymolysis liquid;
(3) adsorption: adsorbing the enzymolysis liquid in step (2) to obtain a mixture liquid;
(4) filtration and purification: removing an adsorbent and denatured hyaluronidase from the mixture liquid in step (3), and filtering to obtain a solution of pure small-molecule hyaluronic acid or salts thereof; and
(5) low-temperature spray drying: making the solution of the small-molecule hyaluronic acid or salts thereof in step (4) subject to low-temperature spray drying to obtain a solid powder of the small-molecule hyaluronic acid or salts thereof having a desired molecular weight.

2. The method for preparing small-molecule hyaluronic acid or salts thereof according to claim 1, wherein in the step (1), the hyaluronidase is first added to the purified water, stirring is started, a pH is adjusted to 5-8, a temperature is 30-40 °C, the solid powder of the hyaluronic acid or salts thereof is slowly added thereto so that a mass volume concentration reaches 50-100 g/L, 0.5-1.5 h after a degradation reaction, the solid powder of the hyaluronic acid or salts thereof continues to be added slowly so that the mass volume concentration reaches 100-200 g/L, and enzymolysis continues for 5-8 h.

3. The method for preparing small-molecule hyaluronic acid or salts thereof according to claim 1, wherein in the step (1), an addition amount of the hyaluronidase is 10²-10⁵ IU per gram of the hyaluronic acid or salts thereof.

4. The method for preparing small-molecule hyaluronic acid or salts thereof according to claim 1, wherein in the step (2), a condition of denaturation and inactivation of the hyaluronidase is: heating the enzymolysis liquid to 45-80 °C and maintaining for 5-60 min.

5. The method for preparing small-molecule hyaluronic acid or salts thereof according to claim 1, wherein in the step (3), adsorption condition of the enzymolysis liquid is: 0.01%-1% activated carbon, and adsorption for 30-90 min, and in the step (4), a cellulose filter membrane having a pore diameter of 0.22 µm is used to perform the filtration.

6. The method for preparing small-molecule hyaluronic acid or salts thereof according to claim 2, wherein hyaluronate is sodium hyaluronate, and hydrochloric acid or sodium hydroxide solution is used for a pH adjustment.

7. The method for preparing small-molecule hyaluronic acid or salts thereof according to claim 1, wherein in the step (4), a final concentration of the solution of the small-molecule hyaluronic acid or salts thereof is 10%-20%.

8. The method for preparing small-molecule hyaluronic acid or salts thereof according to claim 1, wherein in step (5), condition of the low-temperature spray drying is: an inlet air temperature of 40 °C-80 °C, an outlet air temperature of 40 °C, and a feed rate of 100 mL/h-300 mL/h.

9. The method for preparing small-molecule hyaluronic acid or salts thereof according to claim 1, wherein the hyaluronic acid or salts thereof used for enzymolysis in the step (1) have a molecular weight of 500 kDa-700 kDa, and the small-molecule hyaluronic acid or salts thereof prepared in the step (5) have a molecular weight of 1 kDa-60 kDa.

10. The method for preparing small-molecule hyaluronic acid or salts thereof according to any one of claims 1-9, wherein a method for producing the hyaluronidase comprises steps of:
(a) inoculating a *Lactobacillus plantarum* CnT012-56 strain into a sterilized seed medium, with a culture temperature of 30 °C-37 °C, a rotation speed of 0-100 rpm, and a culture duration of 8-24 h, to obtain a seed culture solution;
(b) inoculating the seed culture solution in step (a) into a sterilized fermentation medium, with a culture temperature of 30 °C-37 °C, a rotation speed of 0-150 rpm, and a culture duration of 20-48 h, to obtain a hyaluronidase fermentation broth;
(c) centrifuging the hyaluronidase fermentation broth in step (b) to obtain a supernatant;
(d) ultrafiltering hyaluronidase supernatant in step (c) using an ultrafiltration membrane having a pore diameter with molecular weight cutoff of 200 kDa, to obtain a filtrate; and
(e) ultrafiltering the filtrate in step (d) using an ultrafiltration membrane having a pore diameter with molecular weight cutoff of 20 kDa, to remove small-molecule impurities to obtain purified hyaluronidase.

11. The method for preparing small-molecule hyaluronic acid or salts thereof according to claim 10, wherein components of the seed medium in the step (a) are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with a pH of 6.5.

12. The method for preparing small-molecule hyaluronic acid or salts thereof according to any one of claims 10 to 11, wherein components of the fermentation medium in the step (b) are: peptone 10 g/L, yeast extract powder 5 g/L, hyaluronic acid 5 g/L, glucose 3 g/L, sodium chloride 0.1 g/L, ammonium sulfate 2 g/L, ferrous sulfate 0.05 g/L, magnesium sulfate 0.2 g/L, and Tween-80 1 mL, with a pH of 6.5.

13. A small-molecule hyaluronic acid or salts thereof, **characterized in that** the small-molecule hyaluronic acid or salts thereof are prepared by the method for preparing small-molecule hyaluronic acid or salts thereof according to any one of claims 1-12.
